# EUROPÄISCHE PATENTSCHRIFT

(11) **EP 1 842 118 B1**
(45) Veröffentlichungstag und Bekanntmachung des Hinweises auf die Patenterteilung: **20.10.2010**
(21) Anmeldenummer: 05810672.5
(22) Anmeldetag: 02.11.2005
(51) Int. Cl.: G05D 16/20, A61M 16/00

(54) **VERFAHREN UND VORRICHTUNG ZUR DRUCKREGELUNG**
PRESSURE CONTROL METHOD AND DEVICE
PROCEDE ET DISPOSITIF DE REGULATION DE PRESSION

(30) Priorität: 26.01.2005 DE 102005003553
(43) Veröffentlichungstag der Anmeldung: 10.10.2007
(73) Patentinhaber: Weinmann Geräte für Medizin GmbH & Co. KG, 22525 Hamburg (DE)
(72) Erfinder: GÖBEL, Christoph, 22457 Hamburg (DE); HERRMANN, Frank, 23843 Bad Oldesloe (DE)
(74) Vertreter: Klickow, Hans-Henning
(86) Internationale Anmeldenummer: PCT/DE2005/001956
(87) Internationale Veröffentlichungsnummer: WO 2006/079301

(56) Entgegenhaltungen:
- EP-A- 0 549 299
- EP-A- 0 903 160
- US-A- 5 245 995

## Beschreibung

Die Erfindung betrifft ein Verfahren zur Druckregelung in einer Einrichtung zur pneumatischen Versorgung, bei dem von einer Gasquelle eine Gasströmung erzeugt und über eine Verbindungsleitung einer Abnahmestelle zugeführt wird und bei dem ein Ist-Wert des Druckes meßtechnisch erfaßt und von einem Druckregler bei der Druckregelung ausgewertet wird, wobei die meßtechnische Erfassung des Druckes im Bereich einer Verengung der Verbindungsleitung und mit einem räumlichen Abstand zur Abnahmestelle durchgeführt wird.

Die Erfindung betrifft darüber hinaus eine vorrichtung zur Druckregelung in einer Einrichtung zur pneumatischen Versorgung, die eine Gasquelle sowie eine über eine Verbindungsleitung an die Gasquelle angeschlossene Abnahmestelle aufweist sowie die sowohl mit mindestens einem sensor zur Druckerfassung als auch mit einem Druckregler versehen ist und bei der mindestens ein Sensor zur Druckerfassung mit einem räumlichen Abstand zur Abnahmestelle im Bereich einer Verengung der Verbindungsleitung angeordnet ist.

Derartige Verfahren und Vorrichtungen werden beispielsweise zur Gerätesteuerung im Bereich von Beatmungsgeräten eingesetzt. Derartige Beatmungsgeräte sind über eine schlauchartige Verbindungsleitung mit einer als Atemmaske ausgebildeten Abnahmestelle verbunden. Eine Druckerfassung erfolgt entweder über Druckmeßschläuche im Bereich der Atemmaske oder direkt innerhalb des Beatmungsgerätes. Eine Druckmessung unter Verwendung von Druckmeßschläuchen im Bereich der Beatmungsmaske weist den vorteil einer guten Genauigkeit auf. Nachteilig ist jedoch der vergleichsweise große gerätetechnische Aufwand für den Druckmeßschlauch.

Eine Druckmessung unmittelbar im Bereich des Beatmungsgerätes ist zwar konstruktiv einfach zu realisieren, aufgrund von Druckverlusten entlang des Verbindungsschlauches wird jedoch die Regelungsgenauigkeit vermindert. Insbesondere erweist es sich als nachteilig, daß die anfallenden Druckverluste abhängig vom Strömungsvolumen sind.

Die Druckverluste entlang der Verbindungsleitung weisen in der Regel mehrere Komponenten auf. Bei laminaren Strömungen steigt der Druckverlust linear mit der Strömungsgeschwindigkeit bzw. mit dem Volumenstrom. Bei einem Auftreten von turbulenten Strömungen erhöht sich der Druckverlust mit dem Quadrat der Strömungsgeschwindigkeit.

Aus der EP 0 903 160 ist bereits eine Vorrichtung zur Druckregelung in einer Einrichtung zur pneumatischen Versorgung bekannt. Es wird hierbei eine Abnahmestelle über eine Verbindungsleitung an eine Gasquelle angeschlossen. Unter Verwendung eines Sensors zur Druckerfassung erfolgt eine Druckregelung. Der Sensor ist mit einem räumlichen Abstand zur Abnahmestelle im Bereich einer Verengung der Verbindungsleitung angeordnet.

Die US 5,245,995 A beschreibt ebenfalls eine Druckregelung in einer Einrichtung zur pneumatischen Versorgung, bei der von einer Gasquelle eine Gasströmung erzeugt wird. Auch gemäß diesem Stand der Technik erfolgt eine Druckregelung basierend auf einer meßtechnischen Erfassung des Druckes.

Eine weitere Druckregelung in einer Einrichtung zur pneumatischen Versorgung wird in der EP 0 549 299 A beschrieben. Auch hier wird wiederum eine Druckregelung basierend auf einem meßtechnisch erfaßten Druck durchgerührt.

Aufgabe der vorliegenden Erfindung ist es, ein Verfahren und eine Vorrichtung der einleitend genannten Art derart zu verbessern, daß bei einfachem konstruktiven Aufbau eine hohe Genauigkeit der Druckregelung unterstützt wird.

Diese Aufgabe wird erfingdungsgemäß dadurch gelöst, daß die Verengung konstruktiv an die Strömungseigenschaften der Verbindungsleitung angepasst ist, wodurch im Bereich der Verengungsstelle ein reduzierter Druck bereitgestellt wird, der dem Druck im Bereich der Abnahmestelle entspricht.

Durch die Druckmessung im Bereich einer Verengung der Verbindungsleitung wird der physikalische Effekt ausgenutzt, daß durch die erhöhte Strömungsgeschwindigkeit im Bereiche der Verengung ein verminderter Druck vorliegt. Bei einer konstruktiven Anpassung der Verengung an die Strömungseigenschaften der Verbindungsleitung kann konstruktiv im Bereich der Verengungsstelle ein reduzierter Druck bereitgestellt werden, der dem Druck im Bereich der Abnahmestelle entspricht. Aufgrund der gleichartigen Beeinflussung der Druckreduzierung im Bereich der Verengung und der Druckreduzierung im Bereich der Abnahmestelle durch Druckverluste entlang der Verbindungsleitung kann an der Verengungsstelle im wesentlichen der gleiche Druck gemessen werden, der in örtlicher Entfernung zur Verengungsstelle im Bereich der Abnahmestelle auftritt.

Es ist somit regelungstechnisch eine direkte Auswertung des Druckmeßwertes im Bereich der Verengungsstelle ohne Durchführung zusätzlicher rechnerischer Maßnahmen möglich. Insbesondere wird auch eine Minimierung der Reaktionszeit der Regelung auf Druckänderungen erreicht, da Verzögerungen aufgrund des Innenvolumens von verwendeten Druckmeßschläuchen wegfallen.

Ein geringer resultierender Gesamtströmungswiderstand wird dadurch erreicht, daß die Gasströmung vor und nach der Verengung durch Bereiche der Verbindungsleitung mit relativ zueinander im wesentlichen gleicher Querschnittfläche strömt.

Eine besonders optimale Durchführung der Druckregelung unter Berücksichtigung konkret vorliegender Strömungswiderstände wird dadurch erreicht, daß ein sich einstellender Druck in der Verengung an einen Strömungswiderstand des weiteren Bereiches der Verbindungsleitung angepaßt wird.

Eine weitere Verbesserung des Regelungsverhaltens kann dadurch erreicht werden, daß mindestens eine Druckmessung in Strömungsrichtung vor der Verengung durchgeführt wird.

Darüber hinaus ist auch daran gedacht, daß mindestens eine Druckmessung in Strömungsrichtung hinter der Verengung durchgeführt wird.

Eine über die Querschnittfläche der Verbindungsleitung ungleichmäßige Druckverteilung kann dadurch berücksichtigt werden, daß mindestens eine Druckmessung hinsichtlich einer Querschnittfläche des Strömungsweges benachbart zu einer Wandung und mindestens eine weitere Druckmessung hinsichtlich der Querschnittfläche des Strömungsweges in einem relativ zur ersten Druckmessung weiter innen liegenden Bereich durchgeführt wird.

Eine weitere Variante zur Druckregelung besteht darin, daß die Gasströmung hinter der Verengung durch mindestens eine zweite Verengung geleitet wird.

Darüber hinaus ist auch daran gedacht, daß die Gasströmung hinter der Verengung durch mindestens ein Erweiterungssegment geleitet wird.

In den Zeichnungen sind Ausführungsbeispiele der Erfindung schematisch dargestellt. Es zeigen:
- Fig. 1: eine perspektivische Darstellung eines Beat- mungsgerätes mit Verbindungsschlauch zu einer Beatmungsmaske,
- Fig. 2: eine schematische Darstellung einer Druckrege- lung mit Druckerfassung im Bereich einer Veren- gungsstelle des Verbindungsschlauches, oder ei- nes verengten Strömungsabschnittes innerhalb des Gerätes,
- Fig. 3: eine gegenüber Fig. 2 abgewandelte Ausführungs- form mit zusätzlicher Druckmessung in einem Verbindungsbereich zwischen der Verengung und der Beatmungsmaske,
- Fig. 4: eine schematische Darstellung einer Ausfüh- rungsform der Verengung mit sprungartiger Quer- schnittsveränderung,
- Fig. 5: eine gegenüber Fig. 4 abgewandelte Ausführungs- form mit gekrümmt begrenzter Verengung,
- Fig. 6: eine weitere Ausführungsform der Verengung,
- Fig. 7: eine nochmals modifizierte Ausführungsform der Querschnittveränderung,

- Fig. 8: eine Darstellung ähnlich zu Fig. 4 mit zusätz- licher Lokalisierung von Druckmeßstellen,
- Fig. 9: eine Darstellung ähnlich zu Fig. 5 mit zusätz- licher Lokalisierung von Druckmeßstellen,
- Fig. 10: eine Darstellung ähnlich zu Fig. 6 mit zusätz- licher Lokalisierung von Druckmeßstellen,
- Fig. 11: eine Darstellung ähnlich zu Fig. 7 mit zusätz- licher Lokalisierung von Druckmeßstellen,
- Fig. 12: eine schematische Darstellung zur Veranschauli- chung mehrerer Druckmeßstellen mit unterschied- lich weit in die Strömung hineinragenden Druk- kerfassungspunkten,
- Fig.: 13 eine Ausführungsform mit einer der Gasquelle zugewandt angeordneten Druckmeßstelle,
- Fig. 14: eine Ausführungsform mit der Beatmungsmaske zu- gewandt angeordneten Erfassungsstellen,
- Fig. 15: eine Ausführungsform unter Verwendung einer hintereinander angeordneten Querschnittveren- gung und einer Querschnitterweiterung der Ver- bindungsleitung,
- Fig.: 16 eine weitere Ausführungsform einer Verengung mit lokalisierten Druckmeßstellen,
- Fig. 17: eine Abwandlung zur Ausführungsform in Fig. 15 und

- Fig.: 18 eine weitere Ausführungsform mit aufeinander- folgenden Querschnittverengungen sowie lokali- sierten Druckmeßstellen.

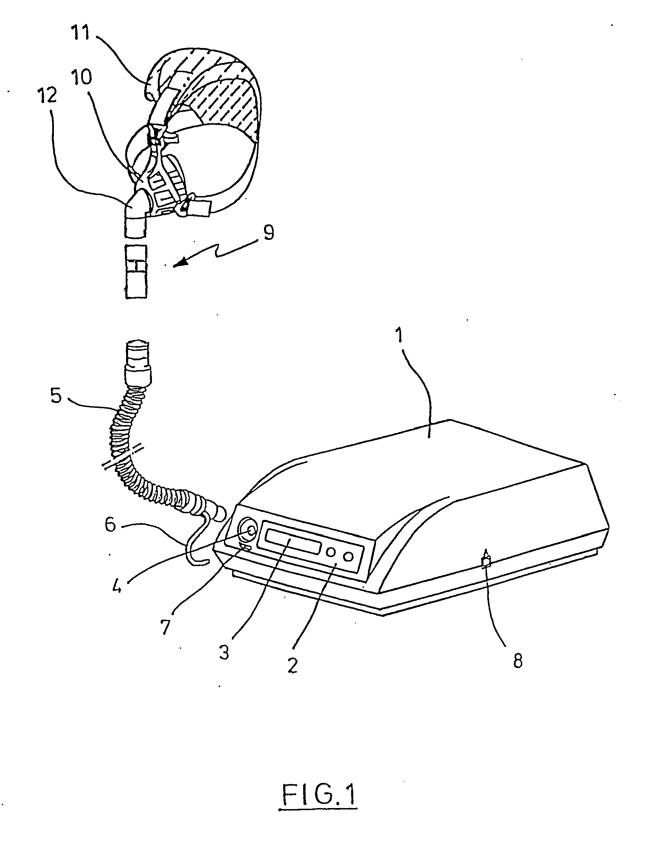

Fig. 1 zeigt den grundsätzlichen Aufbau einer Vorrichtung zur Beatmung. Im Bereich eines Gerätegehäuses (1) mit Bedienfeld (2) sowie Anzeige (3) ist in einem Geräteinnenraum eine Atemgaspumpe angeordnet. Über eine Kopplung (4) wird eine als Verbindungsschlauch ausgebildete Verbindungsleitung (5) angeschlossen. Entlang des Verbindungsschlauches (5) kann ein zusätzlicher Druckmeßschlauch (6) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf.

Im Bereich einer dem Gerätegehäuse (1) abgewandten Ausdehnung der Verbindungsleitung (5) ist ein Ausatmungselement (9) angeordnet. Ebenfalls kann ein Ausatemventil verwendet werden.

Fig. 1 zeigt darüber hinaus eine Beatmungsmaske (10), die als Nasalmaske ausgebildet ist. Eine Fixierung im Bereich eines Kopfes eines Patienten kann über eine Kopfhaube (11) erfolgen. Im Bereich ihrer dem Verbindungsschlauch (5) zugewandten Ausdehnung weist die Beatmungsmaske (10) ein Kupplungselement (12) auf.

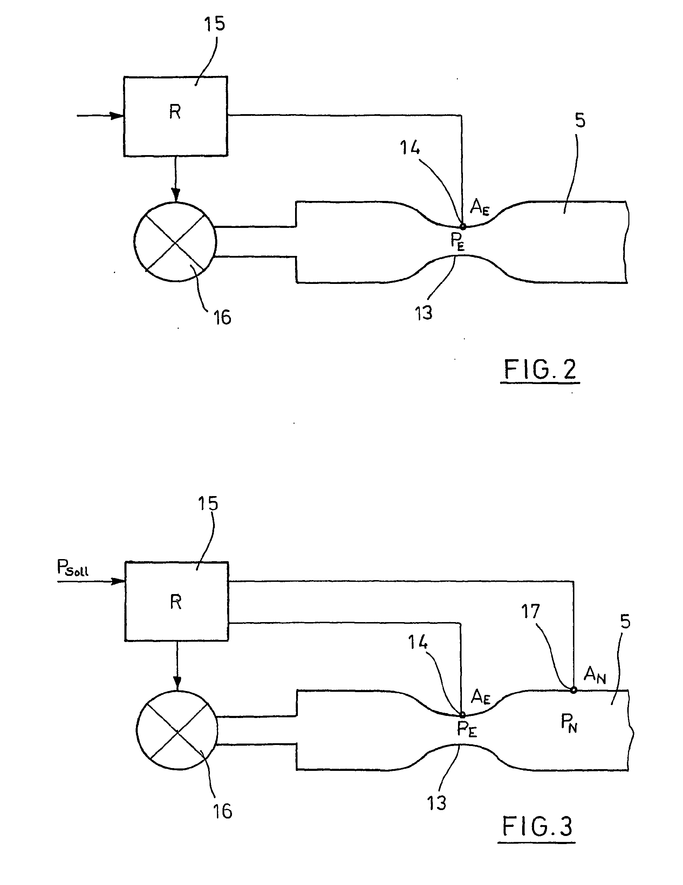

Fig. 2 zeigt die als Verbindungsschlauch ausgebildete Verbindungsleitung (5) mit einer Verengung (13). Im Bereich der Verengung (13) ist ein Sensor (14) zur Drukkerfassung angeordnet. Als Sensor (14) können eine Vielzahl von Einrichtungen zur Druckerfassung verwendet werden. Beispielsweise sind mechanische oder elektrische Druckaufnehmer verwendbar. Ebenfalls ist es denkbar, den Sensor (14) als eine Druckmeßleitung zu realisieren, die den zu messenden Druck in den Bereich eines Signalwandlers weiterleitet. Der Sensor (14) ist an einen Druckregler (15) angeschlossen, der eine die Verbindungsleitung (5) speisende Gasquelle (16) steuert. Die Gasquelle (16) kann beispielsweise als ein elektrisch angetriebenes Gebläse ausgeführt sein.

Gemäß der Ausführungsform in Fig. 3 wird ein zweiter Sensor (17) zur Druckerfassung in einem Bereich der Verbindungsleitung (5) angeordnet, der zwischen der Verengung (13) und der Beatmungsmaske (10) angeordnet ist. Der zweite Sensor (17) erfaßt den Normaldruck innerhalb der Verbindungsleitung (5).

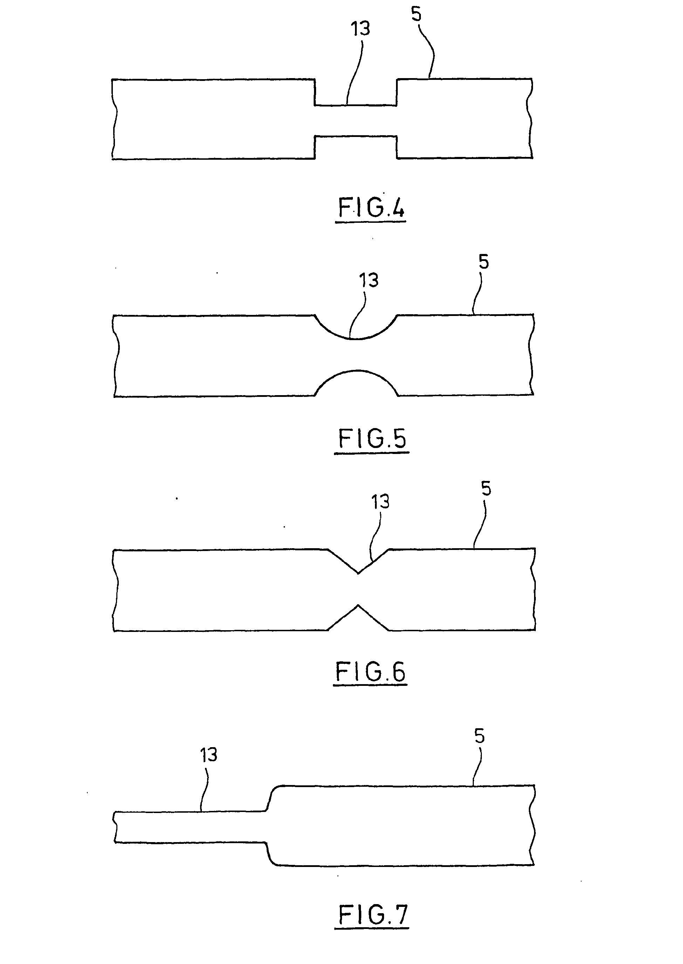

Fig. 4 zeigt eine Ausführungsform der Verengung (13) mit sprungförmiger Veränderung des Strömungsquerschnittes. Gemäß der Ausführungsform in Fig. 5 verläuft eine Kontur der Verengung (13) im wesentlichen Bereich kontinuierlich gewölbt.

Gemäß der Ausführungsform in Fig. 6 ist für die Verengung (13) in Strömungsrichtung zunächst eine im wesentlichen lineare Verminderung des Querschnittes und im Anschluß an eine Stelle eines geringsten Querschnittes eine im wesentlichen lineare Querschnittvergrößerung vorgesehen. Gemäß der Ausführungsform in Fig. 7 erfolgt eine Querschnittveränderung ähnlich wie in Fig. 4, jedoch mit gerundet konturierten Übergangsbereichen entsprechend einen S-Profil.

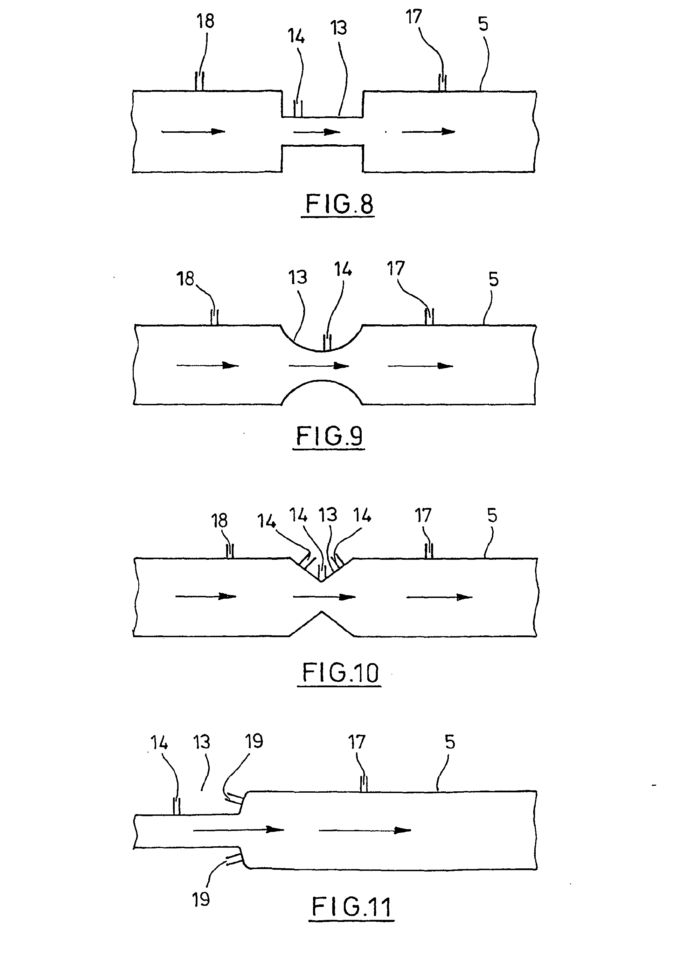

Fig. 8 zeigt eine Ausführungsform ähnlich wie in Fig. 4 mit einem zweiten Sensor (17) im Bereich des der Beatmungsmaske (10) zugewandten Teiles der Verbindungsleitung (5) sowie einem dritten Sensor (18) zur Druckmessung im Bereich eines der Gasquelle (16) zugewandten Teiles der Verbindungsleitung (5).

Fig. 9 zeigt eine Anordnung ähnlich zur Darstellung in Fig. 5 ebenfalls mit einem zweiten Sensor (17) sowie einem dritten Sensor (18).

Bei der Ausführungsform gemäß Fig. 10 sind ausgehend von der Grundkonstruktion in Fig. 6 sowohl zweite und dritte Sensoren (17, 18) als auch drei Sensoren (14) im Bereich der Verengung (13) angeordnet. Der erste der Sensoren (14) befindet sich im Bereich einer zunehmenden Verjüngung der Verengung (13), der zweite Sensor (14) ist im Bereich der kleinsten Querschnittfläche der Verengung (13) angeordnet und der dritte Sensor (14) befindet sich in einem Bereich wieder zunehmender Querschnittfläche der Verengung (13).

Gemäß der Ausführungsform in Fig. 11 sind ausgehend von der Darstellung in Fig. 7 ein Sensor (14) im Bereich der Verengung (13) sowie ein zweiter Sensor (17) im Bereich einer relativ zur Verengung (13) größeren Querschnittfläche der Verbindungsleitung (5) angeordnet. Zusätzliche Sensoren (19) zur Druckerfassung befinden sich im Bereich des Überganges von der Verengung (13) in den weiteren Bereich der verbindungsleitung (5).

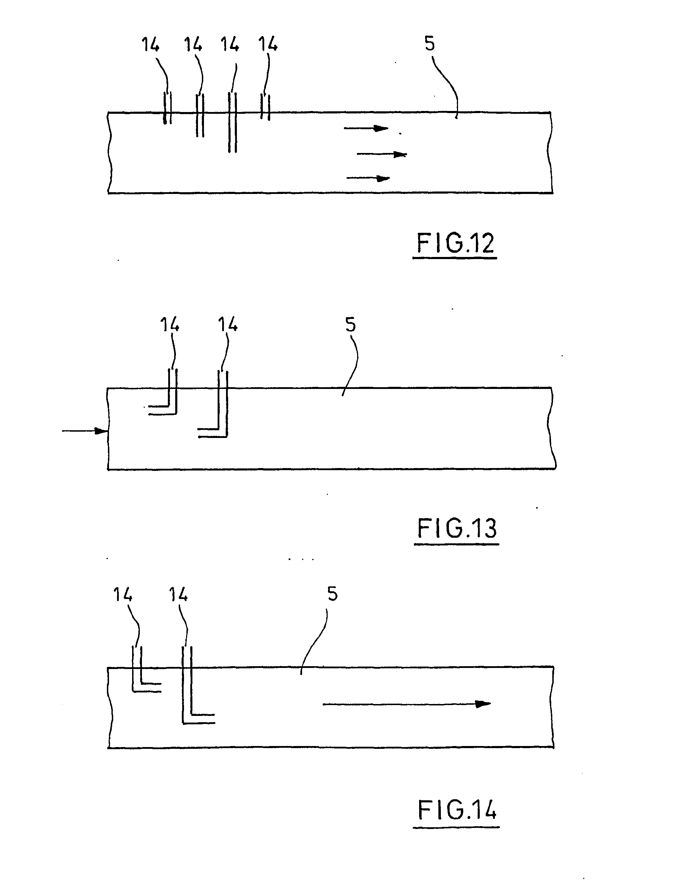

Fig. 12 zeigt eine Ausführungsform zur meßtechnischen Druckerfassung an unterschiedlichen Querschnittpositionen der Verbindungsleitung (5). Eine entsprechende Anordnung kann auch im Bereich der Verengung (13) erfolgen. Durch ein unterschiedlich weites Hineintauchen der Sensoren (14) kann die über die Querschnittfläche der Verbindungsleitung (5) ungleichmäßige Verteilung der Strömungsgeschwindigkeit und damit auch des Druckes berücksichtigt werden. In der Regel strömt das Gas in einem zentralen Bereich der Verbindungsleitung (5) schneller als in einem der Wandung der Verbindungsleitung (5) zugewandten Bereich. Eine Druckmessung ausschließlich in der Nähe der Wandung würde somit zu einem Druckmeßwert führen, der größer als ein bezüglich der Querschnittfläche mittlerer Druck ist, da der statische Druck an der Wandung größer als in der Mitte ist.

Gemäß der Ausführungsform in Fig. 13 werden zur Drukkerfassung Sensoren (14) verwendet, die eine derart abgewinkelte Gestaltung besitzen, daß ein unmittelbar von der Gasströmung beaufschlagter Eingangsbereich der Sensoren (14) der Gasströmung zugewandt angeordnet ist. Bei der Ausführungsform gemäß Fig. 13 ist dieser Erfassungsbereich der Sensoren (14) der von der Gasquelle (16) ausgehenden Gasströmung zugewandt.

Gemäß der Ausführungsform in Fig. 14 erfolgt eine Anordnung ähnlich wie in Fig. 13, ein Erfassungsbereich der Sensoren (14) ist jedoch einer von der Beatmungsmaske (10) ausgehenden Rückströmung zugewandt.

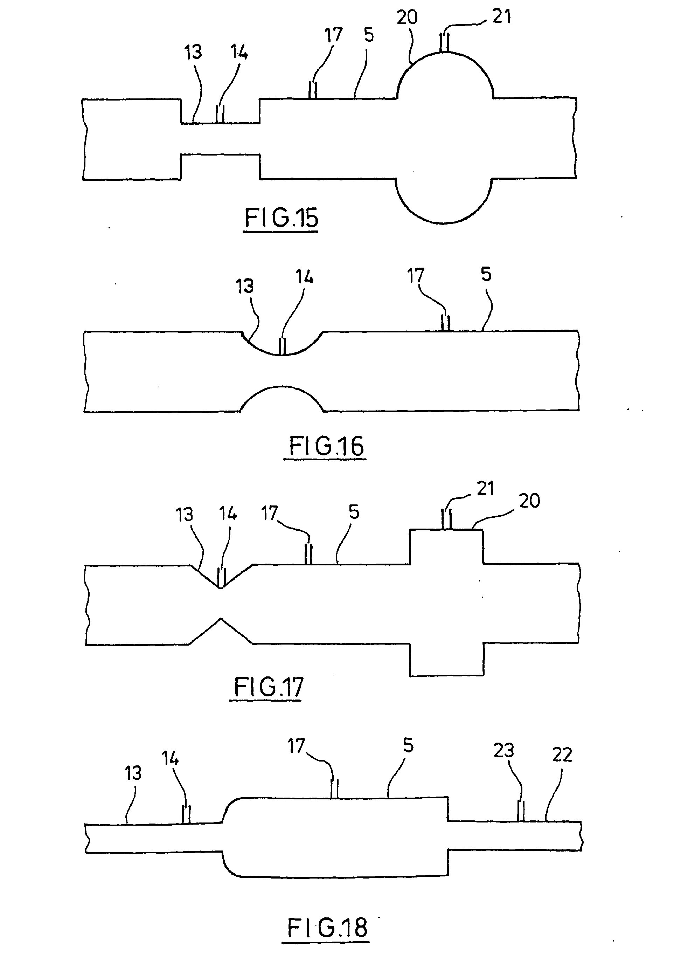

Gemäß der Ausführungsform in Fig. 15 ist in Strömungsrichtung hinter der Verengung (13) und hinter einem Segment der Verbindungsleitung (5) mit Normalquerschnitt ein Erweiterungssegment (20) nachgeordnet. Das Erweiterungssegment (20) ist mit einem Drucksensor (21) ausgestattet. Ein Wandungsverlauf im Bereich des Erweiterungssegmentes (20) erfolgt vorzugsweise mit einer gerundeten Kontur.

Fig. 16 zeigt eine Abwandlung zur Ausführungsform in Fig. 9, jedoch ohne Verwendung des dritten Sensors (18). Gemäß der Ausführungsform in Fig. 17 erfolgt eine Abwandlung der Ausführungsform in Fig. 15. Die Verengung (13) ist hier ähnlich zur Ausführungsform in Fig. 6 ausgebildet. Das Erweiterungssegment (20) ist bei dieser Ausführungsform mit einer sprungförmigen Querschnittveränderung sowohl hinsichtlich der Erweiterung als auch hinsichtlich der anschließenden Verengung versehen.

Bei der Ausführungsform gemäß Fig. 18 ist hinter einer Reihenschaltung der Verengung (13) und eines mit Normalquerschnitt versehenen Segmentes der Verbindungsleitung (5) eine zweite Verengung (22) mit Drucksensor (23) angeordnet. Im Bereich des zwischen den Verengungen (13, 22) angeordneten Segmentes der Verbindungsleitung (5) können die Sensoren (17) in unterschiedlicher Ausführungsform angeordnet werden. Insbesondere ist auch an eine Verwendung von Sensoren gedacht, die im Zusammenhang mit den Figuren 13 und 14 bereits erläutert wurden.

## Patentansprüche

1. Verfahren zur Druckregelung in einer Einrichtung zur pneumatischen Versorgung, bei dem von einer Gasquelle (16) eine Gasströmung erzeugt und über eine Verbindungsleitung (5) einer Abnahmestelle (10) zugeführt wird und bei dem ein Ist-Wert des Druckes meßtechnisch erfaßt und von einem Druckregler (15) bei der Druckregelung ausgewertet wird, wobei die meßtechnische Erfassung des Drukkes im Bereich einer Verengung (13) der Verbindungsleitung (5) und mit einem räumlichen Abstand zur Abnahmestelle (10) durchgeführt wird, **dadurch gekennzeichnet**, die Verengung (13) Konstruktiv an die Strömungs-eigenschaften der Verbindungsleitung (5) angepasst ist, wodurch im Bereich der Verengungsstelle (13) ein reduzierter Druck bereitgestellt wird, der dem Druck im Bereich der Abnahmestelle (10) entspricht.

2. Verfahren nach Anspruch 1, **dadurch gekennzeichnet, daß** die Gasströmung vor und nach der Verengung (13) durch Bereiche der Verbindungsleitung (5) mit relativ zueinander im wesentlichen gleicher Querschnittfläche strömt.

3. Verfahren nach einem der Ansprüche 1 oder 2, **dadurch gekenntzeichnet, daß** mindestens eine Druckmessung in Strömungsrichtung vor der verengung (13) durchgeführt wird.

4. Verfahren nach einem der Ansprüche 1 oder 2, **dadurch gekennzeichnet, daß** mindestens eine Druckmessung in Strömungsrichtung hinter der Verengung (13) durchgeführt wird.

5. Verfahren nach einem der Ansprüche 1 bis 4, **dadurch gekennzeichnet, daß** mindestens eine Druckmessung hinsichtlich einer Querschnittfläche des Strömungsweges benachbart zu einer Wandung und mindestens eine weitere Druckmessung hinsichtlich der Querschnittfläche des Strömungsweges in einem relativ zur ersten Druckmessung weiter innen liegenden Bereich durchgeführt wird.

6. Verfahren nach einem der Ansprüche 1 bis 5, **dadurch gekennzeichnet, daß** die Gasströmung hinter der verengung (13) durch mindestens eine zweite Verengung (22) geleitet wird.

7. Verfahren nach einem der Ansprüche 1 bis 6, **dadurch gekennzeichnet, daß** die Gasströmung hinter der Verengung (13) durch mindestens ein Erweiterungssegment (20) geleitet wird.

8. Vorrichtung zur Druckregelung in einer Einrichtung zur pneumatischen Versorgung, die eine Gasquelle (16) sowie eine über eine Verbindungsleitung (5) an die Gasquelle (16) angeschlossene Abnahmestelle (10) aufweist sowie die sowohl mit mindestens einem Sensor zur Druckerfassung (14, 17, 18) als auch mit einem Druckregler (15) versehen ist, wobei der Sensor zur Druckerfassung (14, 17, 18) mit einem räumlichen Abstand zur Abnahmestelle (10) im Bereich einer Verengung (13) der Verbindungsleitung (5) angeordnet ist, **dadurch gekennzeichnet, daß** die verengung (13) konstruktiv an die Strömungseigenschaften der verbindungsleitung (5) angepasst ist, wodurch im Bereich der Verengungsstelle (13) ein reduzierter Druck bereitgestellt wird, der dem Druck im Bereich der Abnahmestelle (10) entspricht.

9. vorrichtung nach Anspruch 8, **dadurch gekennzeichnet, daß** die verbindungsleitung (5) in Strömungsrichtung vor und hinter der Verengung (13) eine im wesentlichen gleiche Querschnittfläche aufweist.

10. Vorrichtung nach einem der Ansprüche 8 bis 9, **dadurch gekennzeichnet, daß** eine Wandung der Verbindungsleitung (5) im Bereich der Verengung (13) einen im wesentlichen stetigen Verlauf aufweist.

11. Vorrichtung nach einem der Ansprüche 8 bis 9. **dadurch gekennzeichnet, daß** eine Wandung der Verbindungsleitung (5) im Bereich der Verengung (13) einen im wesentlichen unstetigen Verlauf aufweist.

12. Vorrichtung nach einem der Ansprüche 8 bis 11, **dadurch gekennzeichnet, daß** mindestens ein Sensor zur Druckerfassung in Strömungsrichtung vor der verengung (13) angeordnet ist.

13. vorrichtung nach einem der Ansprüche 8 bis 11, **dadurch gekennzeichnet, daß** mindestens ein Sensor zur Druckerfassung in Strömungsrichtung hinter der Verengung (13) angeordnet ist.

14. Vorrichtung nach einem der Ansprüche 8 bis 13, **dadurch gekennzeichnet, daß** mindestens ein Strömungssensor in einem Randbereich der Querschnittfläche der Verbindungsleitung (5) und mindestens ein weiterer Drucksensor in einem zentralen Bereich der Strömungsfläche angeordnet ist.

15. Vorrichtung nach einem der Ansprüche 8 bis 14, **dadurch gekennzeichnet, daß** mindestens zwei Sensoren zur Druckerfassung im Bereich der Verengung (13) angeordnet sind.

16. Vorrichtung nach einem der Anspruche, 8 bis 15, **dadurch gekennzeichnet, daß** im Bereich der Verbindungsleitung (5) mindestens zwei Verengungen (13) in Strömungsrichtung hintereinander angeordnet sind.

17. vorrichtung nach einem der Ansprüche 8 bis 16, **dadurch gekennzeichnet, daß** die Verengung (13) in Strömungsrichtung mit mindestens einem Erweiterungssegment (20) in Reihe geschaltet ist.

## Claims

1. Method for pressure control in a device for pneumatic supply, in which a gas flow is produced by a gas source (16) and is supplied to a removal location (10) via a connection line (5), and in which an actual value of the pressure is detected in technical measurement terms and is evaluated by a pressure controller (15) during pressure control, the detection of the pressure in technical measurement terms being carried out in the region of a narrowed location (13) of the connection line (5) and at a spatial distance from the removal location (10), **characterised in that** the narrowed location (13) is structurally adapted to the flow properties of the connection line (5), whereby there is provided in the region of the narrowed location (13) a reduced pressure which corresponds to the pressure in the region of the removal location (10).

2. Method according to claim 1, **characterised in that** the gas flow flows, upstream and downstream of the narrowed location (13), through regions of the connection line (5) having cross-sectional surface-areas which are substantially identical relative to each other.

3. Method according to either claim 1 or claim 2, **characterised in that** there is carried out at least one pressure measurement upstream of the narrowed location (13) in the flow direction.

4. Method according to either claim 1 or claim 2, **characterised in that** there is carried out at least one pressure measurement downstream of the narrowed location (13) in the flow direction.

5. Method according to any one of claims 1 to 4, **characterised in that** there is carried out at least one pressure measurement with regard to a cross-sectional surface-area of the flow path adjacent to a wall and at least one other pressure measurement with regard to the cross-sectional surface-area of the flow path in a region located further inwards relative to the first pressure measurement.

6. Method according to any one of claims 1 to 5, **characterised in that** the gas flow downstream of the narrowed location (13) is directed through at least a second narrowed location (22).

7. Method according to any one of claims 1 to 6, **characterised in that** the gas flow downstream of the narrowed location (13) is directed through at least one widened segment (20).

8. Device for pressure control in a pneumatic supply device, which has a gas source (16) and a removal location (10) connected to the gas source (16) via a connection line (5) and which is provided both with at least one sensor (14, 17, 18) for detecting pressure and with a pressure controller (15), the sensor (14, 17, 18) for detecting pressure being arranged at a spatial distance from the removal location (10) in the region of a narrowed location (13) of the connection line (5), **characterised in that** the narrowed location (13) is structurally adapted to the flow properties of the connection line (5), whereby there is provided in the region of the narrowed location (13) a reduced pressure which corresponds to the pressure in the region of the removal location (10).

9. Device according to claim 8, **characterised in that** the connection line (5) has a substantially identical cross-sectional surface-area upstream and downstream of the narrowed location (13) in the flow direction.

10. Device according to either claim 8 or claim 9, **characterised in that** a wall of the connection line (5) has a substantially constant progression in the region of the narrowed location (13).

11. Device according to either claim 8 or claim 9, **characterised in that** a wall of the connection line (5) has a substantially non-constant progression in the region of the narrowed location (13).

12. Device according to any one of claims 8 to 11, **characterised in that** at least one sensor for detecting pressure is arranged upstream of the narrowed location (13) in the flow direction.

13. Device according to any one of claims 8 to 11, **characterised in that** at least one sensor for detecting pressure is arranged downstream of the narrowed location (13) in the flow direction.

14. Device according to any one of claims 8 to 13, **characterised in that** at least one flow sensor is arranged in a peripheral region of the cross-sectional surface-area of the connection line (5) and at least one other pressure sensor is arranged in a central region of the flow surface-area.

15. Device according to any one of claims 8 to 14, **characterised in that** at least two sensors for detecting pressure are arranged in the region of the narrowed location (13).

16. Device according to any one of claims 8 to 15, **characterised in that** at least two narrowed locations (13) are arranged one behind the other in the flow direction in the region of the connection line (5).

17. Device according to any one of claims 8 to 16, **characterised in that** the narrowed location (13) is connected in series to at least one widened segment (20) in the flow direction.

## Revendications

1. Procédé de régulation de pression dans un système d'alimentation pneumatique, dans lequel un flux de gaz est généré par une source de gaz (16) et est conduit, par l'intermédiaire d'une conduite de raccordement (5), à un point de réception (10), et dans lequel une valeur réelle de pression est détectée de manière métrologique et est évaluée par un régleur de pression (15) lors de la régulation de pression, la détection métrologique de la pression étant effectuée dans la zone d'un rétrécissement (13) de la conduite de raccordement (5) et à distance du point de réception (10), **caractérisé en ce que** la construction du rétrécissement (13) est adaptée aux propriétés d'écoulement de la conduite de raccordement (5), de sorte que l'on dispose, dans la zone du rétrécissement (13), d'une pression réduite, qui correspond à la pression présente dans la zone du point de réception (10).

2. Procédé selon la revendication 1, **caractérisé en ce que** le flux de gaz passe, en amont et en aval du rétrécissement (13), par des portions de la conduite de raccordement (5), qui présentent, les unes par rapport aux autres, une section transversale sensiblement identique.

3. Procédé selon l'une des revendications 1 ou 2, **caractérisé en ce qu'**au moins une mesure de pression est exécutée dans la direction d'écoulement du flux, en amont du rétrécissement (13).

4. Procédé selon l'une des revendications 1 ou 2, **caractérisé en ce qu'**au moins une mesure de pression est exécutée dans la direction d'écoulement du flux, en aval du rétrécissement (13).

5. Procédé selon l'une des revendications 1 à 4, **caractérisé en ce que** sont effectuées au moins une mesure de pression au sujet d'une section transversale de la voie d'écoulement du flux à proximité d'une paroi, et au moins une autre mesure de pression au sujet de la section transversale de la voie d'écoulement du flux dans une zone située plus loin à l'intérieur par rapport à la première mesure de pression.

6. Procédé selon l'une des revendications 1 à 5, **caractérisé en ce que** le flux de gaz est conduit, en aval du rétrécissement (13), à travers au moins un deuxième rétrécissement (22).

7. Procédé selon l'une des revendications 1 à 6, **caractérisé en ce que** flux de gaz est conduit, en aval du rétrécissement (13), à travers au moins un segment d'élargissement (20).

8. Dispositif de régulation de pression dans un système d'alimentation pneumatique, qui comprend une source de gaz (16) ainsi qu'une conduite de raccordement (5), qui est reliée à la source de gaz (16), et qui est équipé d'au moins un détecteur de pression (14, 17, 18) ainsi que d'un régleur de pression (15), le détecteur de pression (14, 17, 18) étant disposé, à distance du point de réception (10), dans la région d'un rétrécissement (13) de la conduite de raccordement (5), **caractérisé en ce que** la construction du rétrécissement (13) est adaptée au propriétés d'écoulement de la conduite de raccordement (5), de sorte que l'on dispose, dans la zone du rétrécissement (13), d'une pression réduite, qui correspond à la pression présente dans la zone du point de réception (10).

9. Dispositif selon la revendication 8, **caractérisé en ce que** la conduite de raccordement (5) présente, dans la direction d'écoulement du flux, en amont et en aval du rétrécissement (13), une coupe transversale sensiblement identique.

10. Dispositif selon l'une des revendications 8 à 9, **caractérisé en ce qu'**une paroi de la conduite de raccordement (5) présente, dans la région du rétrécissement (13), une évolution essentiellement régulière.

11. Dispositif selon l'une des revendications 8 à 9, **caractérisé en ce qu'**une paroi de la conduite de raccordement (5) présente, dans la région du rétrécissement (13), une évolution essentiellement irrégulière.

12. Dispositif selon l'une des revendications 8 à 11, **caractérisé en ce qu'**au moins un détecteur de pression est disposé dans la direction d'écoulement du flux, en amont du rétrécissement (13).

13. Dispositif selon l'une des revendications 8 à 11, **caractérisé en ce qu'**au moins un détecteur de pression est disposé dans la direction d'écoulement du flux, en aval du rétrécissement (13)

14. Dispositif selon l'une des revendications 8 à 13, **caractérisé en ce qu'**au moins un détecteur de flux est disposé dans une zone marginale de la section transversale de la conduite de raccordement (5) et au moins un autre détecteur de pression est disposé dans une zone centrale de la surface d'écoulement du flux.

15. Dispositif selon l'une des revendications 8 à 14, **caractérisé en ce qu'**au moins deux détecteurs sont disposés dans la zone du rétrécissement (13) pour la détection de la pression.

16. Dispositif selon l'une des revendications 8 à 15, **caractérisé en ce que**, dans la zone de la conduite de raccordement (5), au moins deux rétrécissements (13) sont disposés, l'un derrière l'autre, dans la direction d'écoulement du flux.

17. Dispositif selon l'une des revendications 8 à 16, **caractérisé en ce que** le rétrécissement (13) est connecté en série, dans la direction d'écoulement du flux, avec au moins un segment d'élargissement (20).
